# EUROPEAN PATENT SPECIFICATION

(11) **EP 3 629 829 B1**
(45) Date of publication and mention of the grant of the patent: **27.11.2024**
(21) Application number: 17910901.2
(22) Date of filing: 26.05.2017
(51) Int. Cl.: A45D 33/00, A45D 34/00

(54) **COSMETIC SUBSTRATE COMPRISING CRIMPED FIBERS**
KOSMETISCHES SUBSTRAT MIT GEKRÄUSELTEN FASERN
SUBSTRAT COSMÉTIQUE COMPRENANT DES FIBRES FRISÉES

(43) Date of publication of application: 08.04.2020
(73) Proprietor: ELC Management LLC, Melville, NY 11747 (US)
(72) Inventor: QUE, Chuzhen, Minhang District, Shanghai 201199 (CN); SHEN, Tao, Shanghai 200050 (CN); JIN, Jing, Pudong District, Shanghai 201318 (CN); CHENG, Jing, Pudong District, Shanghai 200127 (CN); LIN, Gang, Pudong District, Shanghai 200135 (CN); YAO, Yiming, Changning District, Shanghai 200051 (CN); CORREIA CALDEIRA, Pedro Manuel, Geel 2440 (BE); HAYASHI, Hiroshi, Machida-shi, Tokyo 194-0023 (JP); PAZOS, Marta Paz, Atlanta, GA 30309 (US); IYER, Sushil, Astoria, NY 11106 (US)
(74) Representative: Novagraaf Group
(86) International application number: PCT/CN2017/086096
(87) International publication number: WO 2018/214148

(56) References cited:
- EP-A1- 3 434 142
- WO-A1-2017/016608
- CN-A- 104 379 646
- CN-A- 105 658 108
- CN-A- 106 038 325
- JP-A- 2013 170 340
- JP-A- 2017 046 740

## Description

### FIELD OF THE INVENTION

The present invention relates to a cosmetic container which provides a non-woven substrate and an applicator, which may be used for the application of cosmetic or personal care compositions to the skin or hair.

### BACKGROUND OF THE INVENTION

Cosmetic foundations are typically contained within a compact. The cosmetic container includes a body and a lid, and within the body is a liquid cosmetic container for holding a substrate for holding a liquid cosmetic composition. Typical substrates for this purpose are conventionally formed from polyethylene foam, polyurethane foam, sponge rubber or foam. These materials are used for holding a cosmetic composition to maintain stable cosmetic.

However, polyether polyurethane foam, for example, suffers from many drawbacks. In one instance, polyether polyurethane foam is limited to cosmetic compositions having a rather narrow viscosity profile. Products that are too thin are not retained by the foam, and product that are too thick can't effectively be injected into the foam. In another instance, certain formulations cannot be injected into polyether polyurethane foam, nitrile butyl rubber, and similar materials because they are incompatible with them. This drawback is quite frequent in the context of organic sunscreen actives. The aforementioned materials also have a tendency to absorb the UV actives, resulting in products which do not exhibit sufficient SPF protection upon use.

In order to address the aforementioned drawbacks, alternative materials, such as synthetic microfiber nonwoven fibers have been employed as substrates for cosmetic fibers. However, nonwoven fibers tend to suffer from a lack of resilience, as compared to polyether polyurethane foam. Also, each of the known substrate executions tend to suffer from product loss due to leakage at the peripheral surface of the substrate. Therefore, known nonwoven fiber configurations are less capable of absorbing, retaining, and uniformly dispense cosmetic compositions from such substrates. Therefore, there is an ongoing need for a cosmetic substrate which avoids the drawbacks of polyether polyurethane foam, which maintaining resilience, absorbency, and uniform dispensing of cosmetic compositions.

WO 2017/016608 A1 discloses an impregnated cosmetic article comprising a screen net and fiber carrier impregnated with a cosmetic composition. JP 2017 046740 A discloses a cosmetic-containing seat material formed by at least three layers of crimped fibers.

### SUMMARY OF THE INVENTION

The present invention relates to a cosmetic container as defined in claim 1. Preferred features of the invention are set out in the dependent claims.

### BRIEF DESCRIPTION OF THE DRAWINGS

FIG. 1 is a photograph showing two cosmetic substrates, one of the substrates including a silicone substantially liquid-impermeable peripheral surface and the other substrate without a liquid-impermeable peripheral surface.
FIG. 2 is a photograph showing a cosmetic liquid leaking from the peripheral edge of a cosmetic substrate which does not include a substantially liquid-impermeable peripheral surface.
FIG. 3 is a photograph showing a cosmetic substrate with a substantially liquid-impermeable peripheral edge as it is compressed.

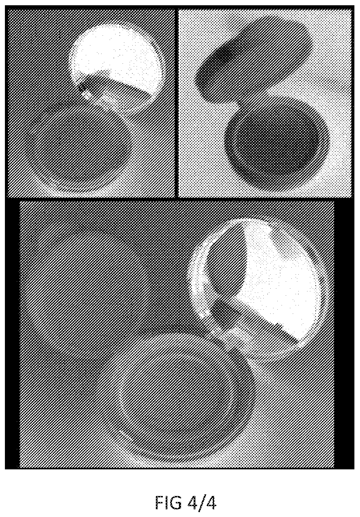

FIG. 4 is a set of photographs showing a cosmetic container containing a compact cosmetic product including a microfiber substrate.

### DETAILED DESCRIPTION OF THE INVENTION

While the specification concludes with claims that particularly point out and distinctly claim the invention, it is believed the present invention will be better understood from the following description.

All percentages, parts and ratios are based upon the total weight of the compositions of the present invention, unless otherwise specified. All such weights as they pertain to listed ingredients are based on the active level, and, therefore, do not include solvents or by-products that may be included in commercially available materials, unless otherwise specified. The term "weight percent" may be denoted as "wt.%" herein.

All molecular weights as used herein are weight average molecular weights expressed as grams/mole, unless otherwise specified.
Herein, "comprising" means that other steps and other ingredients which do not affect the end result can be added. This term encompasses the terms "consisting of" and "consisting essentially of". The compositions and methods/processes of the present invention can comprise, consist of, and consist essentially of the essential elements and limitations of the invention described herein, as well as any of the additional or optional ingredients, components, steps, or limitations described herein.

The term "charge density", as used herein, refers to the ratio of the number of positive charges on a monomeric unit of which a polymer is comprised to the molecular weight of said monomeric unit. The charge density multiplied by the polymer molecular weight determines the number of positively charged sites on a given polymer chain.

The term "microfiber", as used herein, refers to fibers having a decitex value of less than about 50. Preferably, the microfibers have a decitex value of less than about 40, and more preferably less than about 30, and most preferably less than about 15. The microfibers also have a decitex value which is greater than about 0.5, and more preferably greater than about 1.0, and most preferably greater than about 1.5.

The term "polymer" as used herein shall include materials whether made by polymerization of one type of monomer or made by two (i.e., copolymers) or more types of monomers.

The term "solid particle" as used herein means a particle that is not a liquid or a gas.

The term "water-soluble" as used herein, means that the polymer is soluble in water in the present composition. In general, the polymer should be soluble at 25°C at a concentration of at least 0.1% by weight of the water solvent, preferably at least 1%, more preferably at least 5%, most preferably at least 15%.

The term "water-insoluble" as used herein, means that a compound is not soluble in water in the present composition. Thus, the compound is not miscible with water.

The personal care compositions which may be used in the present invention may comprise a cationically modified starch polymer, an anionic surfactant system, and a cosmetically acceptable medium. Further preferred or optional components, are described in detail hereinafter.

### Crimped Microfiber Web

The present invention comprises at least one substrate comprising a web of crimped microfibers.

Webs of the invention are useful for other purposes also, especially where the presence of microfibers, with the special properties provided by them, in combination with loft and moderate density offers a special advantage.

Crimped fibers, i.e. having a continuous wavy, curly, or jagged character along their length, are available in several different forms. Three representative types of known crimped fibers are a generally planar, regularly crimped fiber such as prepared by crimping the fibers with a sawtooth gear; a randomly crimped (random as to the plane in which an undulation occurs and as to the spacing and amplitude of the crimp) such as prepared in a stuffing box; and a helically crimped fiber. Three-dimensional fibers generally encourage greater loftiness in a web of the invention. However, good webs of the invention can be produced from fibers having any of the known types of crimp.

The number of crimps i.e. complete waves or cycles per unit of length can vary rather widely. In general the greater the number of crimps per centimeter (measured by placing a sample fiber between two glass plates, counting the number of complete waves or cycles over a 3-centimeter span, and then dividing that number by 3), the greater the loft of the web.

Crimped fibers also vary in the amplitude or depth of their crimp. Although amplitude of crimp is difficult to uniformly characterize in numerical values because of the random nature of many fibers, an indication of amplitude is given by percent crimp. The latter quantity is defined as the difference between the uncrimped length of the fiber (measured after fully straightening a sample fiber) and the crimped length (measured by suspending the sample fiber with a weight attached to one end equal to 2 milligrams per decitex of the fiber, which straightens the large-radius bends of the fiber) divided by the crimped length and multiplied by 100. Bulking fibers used in the present invention generally exhibit an average percent crimp of at least about 15 percent, and preferably at least about 25 percent.

Cimped fibers according to the description above are commercially available from, for example, Fiber Innovation Technology.

### Microfiber Materials

The microfibers may be formed from natural or synthetic fibers, or from combinations thereof.

Suitable synthetic fibers may include, for example, polyester, thermoplastic elastomers and the like. Particularly preferable synthetic fibers include, for example, Polyethylene(PE), Polypropylene(PP), Polymethyl Methacrylate (PET), Polybutene-1(PB-1), and mixtures thereof.

Polyesters include, for example, polyethylene terephthalate, polytrimethylene terephthalate, polybutylene terephthalate, poly- hexamethylene terephthalate, polytetramethylene, poly 1,4- dimethyl cyclohexane terephthalate, but such poly- hydro lactone, the invention, is not limited to such exemplary only. These polyesters are, respectively may be used alone, in combination with 2 or more kinds may be. Among these thermoplastic polyester is, outstanding resistance, polyethylene terephthalate, polytrimethylene terephthalate, polybutylene terephthalate and poly- hexamethylene terephthalate is preferred.

Thermoplastic elastomers include, for example, polyester-based elastomer, a polyurethane-based elastomer and the like. In all of these, from the viewpoint of improving light resistance of the substrate of a cosmetic of the present invention, polyester-based elastomer is preferred.

Polyester elastomers include, for example, polyesters such as polyethylene terephthalate-based elastomers based elastomer, and a polyester hard segment, a poly (alkylene oxide) glycol polyether soft segments ester block copolymer - and the like. Polyether - ester block copolymer may, for example, a poly (alkylene oxide) glycol and dicarboxylic acids and diols can be prepared by reacting.

The dicarboxylic acids include, for example, phthalic acid o -, m - phthalic acid, terephthalic acid, naphthalene - 2, 6 - dicarboxylic acid, naphthalene - 2, 7 - dicarboxylic acid, diphenyl - 4, 4' - dicarboxylic acid, acid, 3 - sodium sulfoisophthalic acid or an aromatic dicarboxylic acid, 1, 4 - cyclohexanedicarboxylic acid or alicyclic dicarboxylic acid, succinic acid, oxalic acid, adipic acid, sebacic acid, aliphatic dicarboxylic acid such as dimer acid and the like, the present invention is, is not limited to such exemplary only. These dicarboxylic acid, may also be used each alone, 2 or more types may be combined.

Diols may include, for example, ethylene glycol, trimethylene glycol, tetramethylene glycol, penta- methylene glycol, hexamethylene glycol, 1, 4 - butanediol, neopentyl glycol, aliphatic diol such as deca- methylene glycol, 1, 1 - cyclohexane dimethanol, 1, 4 - cyclohexanedimethanol, or alicyclic diols such as tricyclodecanedimethanol include, the invention, is not limited to such exemplary only. These diols are, respectively may be used alone, in combination with 2 or more types may be.

The poly (alkylene oxide) glycols include, for example, polyethylene glycol, poly (1, 2 - propylene oxide) glycol, poly (1, 3 - propylene oxide) glycol, poly (tetramethylene oxide) glycol, ethylene oxide - propylene oxide copolymer, ethylene oxide-tetrahydrofuran copolymer - and the like, according to the present invention, is not limited to such exemplary only. The poly (alkylene oxide) glycols are, respectively may be used alone, in combination with 2 or more types may be. The poly (alkylene oxide) glycol having a number average molecular weight, generally, about 400 - 5000 is preferred.

Polyurethane elastomers are, for example, polyol and diisocyanate obtained by polymerization in the presence of chain extender.

As the diisocyanate, for example, diphenylmethane diisocyanate, tolylene diisocyanate, isophorone diisocyanate, hydrogenated diphenylmethane diisocyanate, xylylene diisocyanate, such as hexamethylene diisocyanate and the like wherein the molecular weight of 500 or less, the present invention is, not limited to such exemplary only. These diisocyanates may be, may also be used each alone, 2 or more kinds may be used.

Chain extending agents include, for example, ethyleneglycol, aminoalcohols, bis hydroxy ethoxy benzene, 1, 4 - butanediol and the like, according to the present invention, is not limited to such exemplary only. The chain extension agent, may also be used each alone, 2 or more kinds may be used.

The microfibers may be formed from one or more types of fibers, for example, by mono or co-extruding the fiber materials. In the case of composite fibers, the individual fibers may be combined according to various orientations. For example, the fibers may combined as a sheath-core type composite fiber, side-by-side type conjugate fiber, island type composite fiber and the like. In all of these, from the viewpoint of efficiently integrating a contact between fibers, sheath-core type composite fiber is preferably, crimping is generated by heating, preferably by-side type conjugate fiber of side. Also, in a particular embodiment, the core fiber of the sheath-core-type configuration are extruded such that they oriented substantially off-center, in a so-called "eccentric sheath-core" configuration, from the radial center of the sheath-core composite fibers. This configuration may assist facilitation of a crimped fiber structure.

In a sheath-core type composite fiber, a core component of the bicomponent fiber are preferably not exposed to the surface. Furthermore, the fibers of the sheath core fibers of the radial center of the radial direction with the center of the more preferred. The sheath-core type composite fiber, concentric sheath-core structure may be formed, eccentric core-sheath-like structure may be formed. The eccentric core-sheath composite fiber having a core-sheath structure is formed in a cylindrical structure, resulting from the heating and crimping. The sectional shape of the core-sheath type conjugate fibers include, for example, circular, triangular, square or polygonal, oval, elliptical and the like, according to the present invention, is not limited to such exemplary only. Among these features, circular is preferred. Preferably the ratio of the core component to the sheath component of the conjugate fiber is from about 30:70 - 70:30.

The substrate, comprising the fibers herein, preferably has fibers with a fineness of from about 0.5 - 15 denier, more preferably 1-10 denier.

The composite fibers of the length, of a cosmetic makeup can be efficiently extracted to the outside is taken into the inside of the substrate, the substrate from the viewpoint of mechanical strength of a cosmetic, preferably about 20 - 150 mm.

The composite fibers may be treated with antimicrobial agents or other surface treatments.

Other suitable fibers for use in the substrate herein may include, for example, cotton, hemp, silk or the like natural fibers, regenerated fibers such as rayon, synthetic fibers and the like. For use in cosmetic applications, synthetic fibers are preferred.

The synthetic fibers include, for example, polyethylene terephthalate fibers, polybutylene terephthalate fibers, polyester fibers such as polytrimethylene terephthalate fibers, polyvinyl chloride fibers, polyvinylidene chloride fibers, polypropylene fibers, polyvinyl alcohol fibers, polyamide fibers such as nylon 6, polyimide fibers, polyamide fibers, cellulose acetate fibers and the like, the present invention is, is not limited to such exemplary only. These fibers may be used alone or in combination with multiple types of fibers. In the case of core-sheath type bicomponent fibers, it is particularly preferred to us a PET sheath with a PP core.

In one embodiment, the substrate may comprise multiple layers, each of which may comprise different characteristics from each other such as density, pore size, hardness, material make up, surface energy, surface treatment, resiliency, and the like. In one embodiment, the surface layer of the substrate has a density which is less than at least the base layer of the substrate. In another embodiment, at least one characteristic of each of the intermediate layers between the surface and base layer increases incrementally and progressively from the top layer to the base layer. For example, in one embodiment, each layer from the top layer to the base layer is increasingly dense. It is believed that incrementally increasing the density of each layer, from the top layer to the bottom layer, provides the benefit of preventing "pooling" of an impregnated cosmetic composition at the base layer of the substrate.

### Liquid Impermeable Rim

One problem presented by substrates used for cosmetic applications is that the peripheral surface of the substrate often suffers from leakage and product loss. Therefore, this invention comprises a cosmetic substrate which comprises a peripheral surface, wherein the peripheral surface is substantially liquid-impermeable.

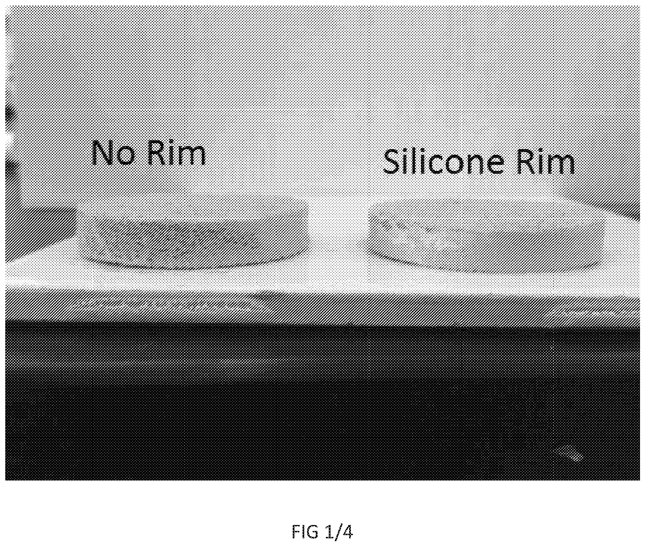

The peripheral surface is heat-sealed, or it is treated with a liquid-impermeable material such as a silicone film. Fig. 1 is a photograph of two substantially identical microfiber substrates. The second substrate comprises a substantially liquid-impermeable rim, provided by a the application of a silicone rim (Macroplast^{®} BK 322). The first substrate is provided without the silicone rim. Both substrates are filled with 11.5g of a cosmetic liquid.

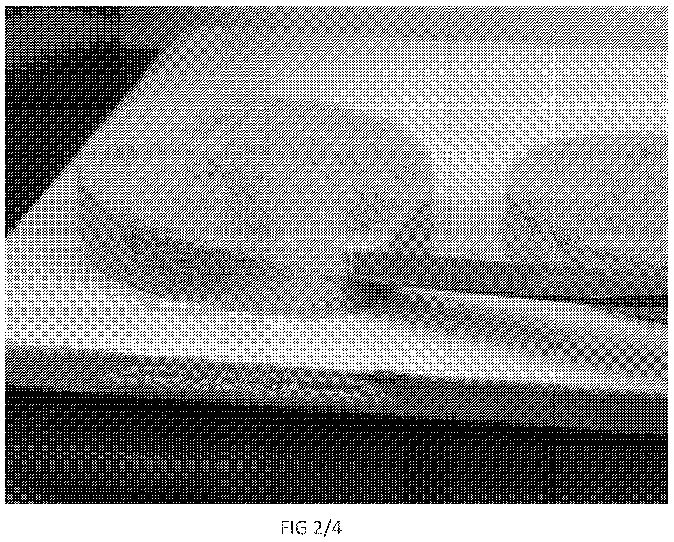

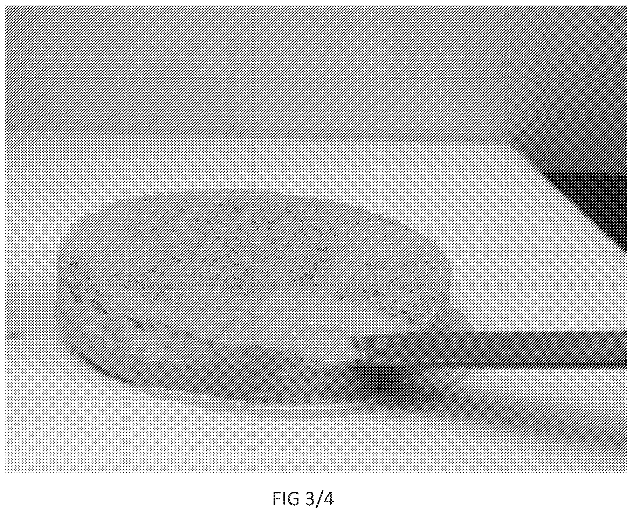

Figs. 2 and 3 are photograph depictions where the edges of each substrate are depressed by about 30%. As is demonstrated in Fig 2, the substrate lacking the liquid-impermeable rim shows product loss from the sides of the substrate. In contrast, Fig. 3 shows the liquid being retained by the substantially liquid-impermeable rim.

While the substrate of the present invention preferably comprises a microfiber web, it is contemplated that other substrates, such as polyurethane polyester or nitrile butyl rubber (NBR) may be treated to include a substantially liquid-impermeable peripheral surface.

### Cosmetic Composition

The cosmetic container and microfiber substrate of the present invention are well-suited for containing a broad variety of cosmetic compositions. Such compositions may include, for example, personal cleansing compositions, makeup compositions, cosmetic treatments, and the like. In particular, the compositions may include physical or chemical sunscreen agents.

Exemplary chemical sunscreen agents include, but are not limited to UVA and UVB sunscreens, such as benzophenones and derivatives thereof (e.g., benzophenone-3, dioxybenzone, sulisobenzone, octabenzone, hydroxy- and/or methoxy-substituted benzophenones, and benzophenonesulfonic acids and salts thereof); salicylic acid derivatives (e.g., ethylene glycol salicylate, triethanolamine salicylate, octyl salicylate, homomenthyl salicylate, and phenyl salicylate); urocanic acid and derivatives thereof (e.g., ethyl urocanate); p-aminobenzoic acid (PABA) and derivatives thereof (e.g., ethyl/isobutyl/glyceryl esters thereof and 2-ethylhexyl p-dimethylaminobenzoate, which is also referred to as octyldimethyl PABA); anthranilates and derivatives thereof (e.g., o-amino-benzoates and various esters of amino-benzoic acid); benzalmalonate derivatives; benzimidazole derivatives; imidazolines; bis-benzazolyl derivatives; dibenzoylmethanes and derivatives thereof; benzoazole/benzodiazole/benzotriazoles and derivatives thereof (e.g., 2-(2-hydroxy-5-methylphenyl) benzotriazole and methylene bis-benzotriazolyl tetramethylbutylphenol, which is commonly referred to as "Tinosorb M"); diesters or polyesters containing diphenylmethylene or 9H-fluorene substitutional groups; 2-phenyl-benzimidazole-5-sulphonic acid (PBSA); 4,4-diarylbutadienes; cinnamates and derivatives thereof (e.g., 2-ethylhexyl-p-methoxycinnamate, octyl-p-methoxycinnamate, umbelliferone, methylumbelliferone, methylaceto-umbelliferone, esculetin, methylesculetin, and daphnetin); camphors and derivatives thereof (e.g., 3-benzylidenecamphor, 4-methylbenzylidenecamphor, polyacrylamidomethyl benzylidenecamphor, benzylidene camphor sulfonic acid, and terephthalylidene dicamphor sulfonic acid, which is commonly referred to as "Encamsule"); triazines and derivatives thereof (e.g., 2,4-bis-{[4-(2-ethyl-hexyloxy)-2-hydroxy]-phenyl}-6-(4-methoxyphenyl)-1,3,5-triazine, which is commonly referred to as "Tinosorb S"); naphthalates and derivatives thereof (e.g., diethylhexyl-2,6-naphthalate); naphtholsulfonates and derivatives thereof (e.g., sodium salts of 2-naphthol-3,6-disulfonic and 2-naphthol-6,8-disulfonic acids); dibenzalacetone and benzalacetonephenone; diphenylbutadienes and derivatives thereof; di-hydroxynaphthoic acid and salts thereof; o- and p-hydroxybiphenyldisulfonates; coumarin derivatives (e.g., 7-hydroxy, 7-methyl, and 3-phenyl derivatives thereof); azoles/diazoles/triazoles and derivatives thereof (e.g., 2-acetyl-3-bromoindazole, phenyl benzoxazole, methyl naphthoxazole, and various aryl benzotriazoles); quinine and derivatives thereof (e.g., bisulfate, sulfate, chloride, oleate, and tannate salts thereof); quinoline and derivatives thereof (e.g., 2-phenylquinoline and 8-hydroxyquinoline salts); tannic acid and derivatives thereof (e.g., hexaethylether derivatives thereof); hydroquinone and derivatives thereof; uric acid and derivatives thereof; vilouric acid and derivatives thereof, and mixtures or combinations thereof. Salts and otherwise neutralized forms of certain acidic sunscreens from the list hereinabove are also useful herein. These organic or chemical sunscreen agents may be used alone or in combination of two or more. In addition, other known animal or vegetable extracts having UV light-absorbing ability may properly be used alone or in combination.

Organic or chemical sunscreen agents that are particularly useful for the practice of the present invention are: 4,4'-t-butyl methoxydibenzoylmethane, 2-ethylhexylsalicylate, 3,3,5-trimethylcyclohexylsalicylate, 2-ethylhexyl p-methoxycinnamate, 2-hydroxy-4-methoxybenzophenone, 2,2-dihydroxy-4-methoxybenzophenone, 2,4-bis-{4-(2-ethyl-hexyloxy)-2-hydroxy]-phenyl}-6-(4-methoxyphenyl)-1,3,5-triazine, methylene bis-benzotriazolyl tetramethylbutylphenol, terephthalylidene dicamphor sulfonic acid, diethylhexyl 2,6-naphthalate, digalloyltrioleate, ethyl 4-[bis(hydroxypropyl)]aminobenzoate, glycerol p-aminobenzoate, methylanthranilate, p-dimethylaminobenzoic acid or aminobenzoate, 2-ethylhexyl p-dimethylaminobenzoate, 2-phenylbenzimidazole-5-sulfonic acid, 2-(p-dimethylaminophenyl)-5-sulfoniobenzoxazoic acid, and mixtures or combinations thereof.

Although organic or chemical sunscreens can be used in the topical composition of the present invention, it is not necessary to include them since the combination of inorganic sunscreen particles (e.g., TiO₂ and/or ZnO) with the alkoxylated diphenylacrylate compound provides a sufficient photo-protective barrier for blocking the harmful UV-A and UV-B radiation. Preferably, the topical composition of the present invention is substantially free of any organic or chemical sunscreens, and more preferably free of dibenzoylmethane derivatives, such as Avobenzone.

### Test Examples 1: Sponge Compatibility test

Sponges of different material were tested with the W/O and O/W emulsion make-up cosmetics compositions. Reference Examples 1 is Polyether PU foam; 2 is Polyester PU foam; 3 is NBR foam; 4 is Non-woven sponge. All kinds of sponges are cut into a cylinder of Ø 48mm * 9mm. After impregnation with formulas for a period, sponges are checked as the TABLE 1.

**TABLE 1**

| | **24 H at RT** | **4 weeks after 50°C** |
|---|---|---|
| **Examples 1 (PU)** | Swell to Ø 55 mm | Swell to Ø 56 mm |
| **Examples 2** | Swell to Ø 50 mm | Cracked |
| **Examples 3 (NBR)** | Swell to Ø 56 mm | Swell to Ø 60 mm |
| **Examples 4 (Nonwoven)** | Maintain appropriate shape | Maintain appropriate shape |

As can be seen in Table 1, in the case of comparative, Examples 1 and 3 will absorb oil matter in formulation and swell a lot, Example 2 does not absorb too much, but it will hydrolysis after a period of storage.

### Test Examples 2: Formulation Stability Test

Sponges of different material in this test were immersed with an O/W formulation with 5 different chemical sun screening. Reference Examples 1 is Polyether PU foam; 3 is NBR foam; 4 is Non-woven sponge. All kinds of sponges are cut into a cylinder of Ø 48mm * 9mm.

After impregnation with formulas for a period, contents of each chemical sun screening was tested. At the same time, pure formulation stored in glass jar without sponge were tested as well. Decreasing of each agent are being shown as below:

| **Examples 1** | | | | |
|---|---|---|---|---|
| | 1M@ 40C | 2M@ 40C | 3M@ 40C | 4M@ 40C |
| Avobenzone | -1.60% | -1.89% | -6.54% | -9.61% |
| Octisalate | -4.58% | -5.57% | -8.96% | -11.93% |
| Octocrylene | -2.53% | -1.72% | -4.26% | -3.80% |
| Oxybenzone | -4.77% | -7.16% | -11.26% | -13.95% |
| Homosalate | -2.51% | -6.30% | -7.19% | -9.95% |

| **Examples 4** | | | | |
|---|---|---|---|---|
| | 1M@ 40C | 2M@ 40C | 3M@ 40C | 4M@ 40C |
| Avobenzone | 1.77% | 0.81 % | -0.96% | |
| Octisalate | 0.58% | -1.91% | -5.56% | |
| Octocrylene | 1.80% | 2.03% | -0.46% | |
| Oxybenzone | 1.44% | -0.30% | -2.83% | |
| Homosalate | 1.16% | -1.27% | -4.11% | |

As we can see from the test examples 2, compare with PU and NBR sponge, Non-woven sponge does not absorb chemical sun screening and keep the formula stable after a period of storage.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the scope of the invention as defined in the appended claims. It is therefore intended to cover in the appended claims all such changes and modifications that are within the scope of this invention.

## Claims

1. A cosmetic container containing at least one non-woven substrate and an applicator, said substrate comprises crimped fibers, wherein said substrate comprises a peripheral surface, **characterized in that** the peripheral surface is substantially liquid-impermeable, wherein the peripheral surface is heat-sealed or treated with a liquid-impermeable material.

2. A cosmetic container according to claim 1, wherein the peripheral surface is treated with a liquid-impermeable material that is a silicone film.

3. A cosmetic container according to claim 1, wherein said non-woven substrate is impregnated with a cosmetic composition.

4. A cosmetic container according to claim 3, wherein the cosmetic composition is a water-in-oil (W/O) type or oil-in-water (O/W) type.

5. A cosmetic container according to claim 3, wherein the cosmetic composition is make-up primer, make-up base, foundation, powder, twin cake, lipstick, lip gloss, eye shadow, eye brow, concealer or blusher.

6. A cosmetic container according to claim 1, wherein said substrate is impregnated with at least one cosmetic composition comprising at least one organic sunscreen agent.

7. A cosmetic container according to claims 1 or 6, wherein the non-woven substrate comprises one or more fibers selected from the group consisting of Polyethylene (PE), Polypropylene (PP), polyethylene terephthalate (PET), Polybutene-1(PB-1), and mixtures thereof.

8. A cosmetic container according to claims 1 or 6, wherein the density of the non-woven substrate is from about 0.015 g/cm³ to about 0.06 g/cm³.

9. A cosmetic container according to claims 1 or 6, wherein the non-woven substrate comprises fibers having an average dtex of from about 3 to about 30.

10. A cosmetic container according to claim 6, wherein the cosmetic composition is a water-in-oil (W/O) type or oil-in-water (O/W) type.

11. A cosmetic container according to claim 6, wherein the cosmetic composition is make-up primer, make-up base, foundation, powder, twin cake, lipstick, lip gloss, eye shadow, eye brow, concealer or blusher.

12. A cosmetic container according to claim 6, wherein said sunscreen agent is selected from the group consisting of Avobenzone, Octisalate, Octocrylene, Oxybenzone, Homosalate, and mixtures thereof

13. A cosmetic container according to claim 1, wherein said substrate comprises fibers with a fineness of from about 0.5 - 15 denier (0.6 - 17 dtex).

## Patentansprüche

1. Kosmetikbehälter, der mindestens ein Vliessubstrat und einen Applikator enthält,
wobei das Substrat gekräuselte Fasern umfasst,
wobei das Substrat eine Umfangsoberfläche umfasst,
**dadurch gekennzeichnet, dass** die Umfangsoberfläche im Wesentlichen flüssigkeitsundurchlässig ist, wobei die Umfangsoberfläche heißversiegelt oder mit einem flüssigkeitsundurchlässigen Material behandelt ist.

2. Kosmetikbehälter nach Anspruch 1, wobei die Umfangsoberfläche mit einem flüssigkeitsundurchlässigen Material behandelt ist, das eine Silikonfolie ist.

3. Kosmetikbehälter nach Anspruch 1, wobei das Vliessubstrat mit einer kosmetischen Zusammensetzung imprägniert ist.

4. Kosmetikbehälter nach Anspruch 3, wobei die kosmetische Zusammensetzung vom Typ Wasser-in-Öl (W/O) oder Öl-in-Wasser (O/W) ist.

5. Kosmetikbehälter nach Anspruch 3, wobei die kosmetische Zusammensetzung Make-up-Primer, Make-up-Basis, Foundation, Puder, Twin Cake, Lippenstift, Lipgloss, Lidschatten, Augenbrauenstift, Concealer oder Rouge ist.

6. Kosmetikbehälter nach Anspruch 1, wobei das Substrat mit mindestens einer kosmetischen Zusammensetzung imprägniert ist, umfassend mindestens ein organisches Sonnenschutzmittel.

7. Kosmetikbehälter nach den Ansprüchen 1 oder 6, wobei das Vliessubstrat eine oder mehrere Fasern umfasst, die aus der Gruppe ausgewählt, bestehend aus Polyethylen (PE), Polypropylen (PP), Polyethylenterephthalat (PET), Polybuten-1 (PB-1) und Mischungen davon.

8. Kosmetikbehälter nach den Ansprüchen 1 oder 6, wobei die Dichte des Vliessubstrats von etwa 0,015 g/cm³ bis etwa 0,06 g/cm³ beträgt.

9. Kosmetikbehälter nach den Ansprüchen 1 oder 6, wobei das Vliessubstrat Fasern umfasst, die ein durchschnittliches dtex von etwa 3 bis etwa 30 aufweisen.

10. Kosmetikbehälter nach Anspruch 6, wobei die kosmetische Zusammensetzung vom Typ Wasser-in-Öl (W/O) oder Öl-in-Wasser (O/W) ist.

11. Kosmetikbehälter nach Anspruch 6, wobei die kosmetische Zusammensetzung Make-up-Primer, Make-up-Basis, Foundation, Puder, Twin Cake, Lippenstift, Lipgloss, Lidschatten, Augenbrauenstift, Concealer oder Rouge ist.

12. Kosmetikbehälter nach Anspruch 6, wobei das Sonnenschutzmittel aus der Gruppe ausgewählt ist, bestehend aus Avobenzon, Octisalat, Octocrylen, Oxybenzon, Homosalat und Mischungen davon.

13. Kosmetikbehälter nach Anspruch 1, wobei das Substrat Fasern mit einer Feinheit von etwa 0,5 bis 15 Denier (0,6 bis 17 dtex) umfasst.

## Revendications

1. Récipient cosmétique contenant au moins un substrat non tissé et un applicateur,
ledit substrat comprenant des fibres frisées,
dans lequel ledit substrat comprend une surface périphérique,
**caractérisé en ce que** la surface périphérique est sensiblement imperméable aux liquides, dans lequel la surface périphérique est scellée à chaud ou traitée avec un matériau imperméable aux liquides.

2. Récipient cosmétique selon la revendication 1, dans lequel la surface périphérique est traitée avec un matériau imperméable aux liquides qui est un film de silicone.

3. Récipient cosmétique selon la revendication 1, dans lequel ledit substrat non tissé est imprégné d'une composition cosmétique.

4. Récipient cosmétique selon la revendication 3, dans lequel la composition cosmétique est de type eau dans huile (E/H) ou huile dans eau (H/E).

5. Récipient cosmétique selon la revendication 3, dans lequel la composition cosmétique est un primer de maquillage, base de maquillage, fond de teint, poudre, poudre compacte, rouge à lèvres, brillant à lèvres, fard à paupières, sourcil, correcteur ou fard à joues.

6. Récipient cosmétique selon la revendication 1, dans lequel ledit substrat est imprégné d'au moins une composition cosmétique comprenant au moins un agent de protection solaire organique.

7. Récipient cosmétique selon les revendications 1 ou 6, dans lequel le substrat non tissé comprend une ou plusieurs fibres choisies dans le groupe constitué de polyéthylène (PE), polypropylène (PP), polyéthylène téréphtalate (PET), polybutène-1 (PB-1), et des mélanges de ceux-ci.

8. Récipient cosmétique selon les revendications 1 ou 6, dans lequel la densité du substrat non tissé est d'environ 0,015 g/cm³ à environ 0,06 g/cm³.

9. Récipient cosmétique selon les revendications 1 ou 6, dans lequel le substrat non tissé comprend des fibres ayant un dtex moyen d'environ 3 à environ 30.

10. Récipient cosmétique selon la revendication 6, dans lequel la composition cosmétique est de type eau dans huile (E/H) ou huile dans eau (H/E).

11. Récipient cosmétique selon la revendication 6, dans lequel la composition cosmétique est un primer de maquillage, base de maquillage, fond de teint, poudre, poudre compacte, rouge à lèvres, brillant à lèvres, fard à paupières, sourcil, correcteur ou fard à joues.

12. Récipient cosmétique selon la revendication 6, dans lequel ledit agent de protection solaire est choisi dans le groupe constitué d'avobenzone, octisalate, octocrylène, oxybenzone, homosalate et des mélanges de ceux-ci.

13. Récipient cosmétique selon la revendication 1, dans lequel ledit substrat comprend des fibres d'une finesse d'environ 0,5 à 15 deniers (0,6 à 17 dtex).
